# EUROPEAN PATENT APPLICATION

(11) **EP 4 163 634 A1**
(43) Date of publication of application: **12.04.2023**
(21) Application number: 21817321.9
(22) Date of filing: 18.05.2021
(51) Int. Cl.: G01N 33/574, C12N 15/12, G01N 27/62

(54) **METHOD AND REAGENT FOR DETECTING PANCREATIC CANCERS**

(30) Priority: 03.06.2020 JP 2020096966
(71) Applicant: Public University Corporation Yokohama City University, Yokohama-shi, Kanagawa 236-0027 (JP); Tosoh Corporation, Yamaguchi 746-8501 (JP)
(72) Inventor: SHIBATA, Wataru, Yokohama-shi, Kanagawa 236-0004 (JP); TERAUCHI, Yasuo, Yokohama-shi, Kanagawa 236-0004 (JP); ENDO, Itaru, Yokohama-shi, Kanagawa 236-0004 (JP); SHIMIZU, Yasuhiro, Yokohama-shi, Kanagawa 236-0004 (JP); OHTAKE, Norihisa, Ayase-shi, Kanagawa 252-1123 (JP); MYOBA, Shohei, Ayase-shi, Kanagawa 252-1123 (JP)
(74) Representative: Novagraaf International SA
(86) International application number: PCT/JP2021/018724
(87) International publication number: WO 2021/246153

(57) **Abstract**

An object of the invention is to provide a method for detecting pancreatic cancer and a reagent that can be used for the method. The method is a method for detecting pancreatic cancer, characterized by comprising measuring the amount of TFPI2 in a body fluid collected from a subject. In addition, an antibody that specifically recognizes the TFPI2 processing polypeptide and intact TFPI2 is included in the reagent for detecting pancreatic cancer.

## Description

### TECHNICAL FIELD

The present invention relates to a method and a reagent for detecting pancreatic cancer in which a tissue factor pathway inhibitor 2 (TFPI2) is measured.

### BACKGROUND ART

About 450,000 people worldwide and about 40,000 people in Japan newly develop pancreatic cancer in one year (GLOBOCAN 2018). The 5-year relative survival rate for pancreatic cancer is 7.9% for males and 7.5% for females, and the 10-year relative survival rate is 4.6% for males and 4.8% for females. Therefore, pancreatic cancer is considered the cancer type with the poorest prognosis.

CA19-9, a typical pancreatic cancer marker, has a high positive rate in pancreatic cancer, biliary tract cancer, and the like, and is considered useful for evaluating the efficacy of chemotherapy and recurrence monitoring. However, there are the following issues: (1) the positive rate for CA19-9 at the early stage is low; (2) colorectal cancer, lung cancer, breast cancer, and the like are also positive for CA19-9; (3) CA19-9 is undetectable in patients with Lewis negative blood type; and (4) CA19-9 also increases in biliary atresia, cholangitis, liver cirrhosis, diabetes, and the like. Other pancreatic cancer biomarkers include DUPAN-2 and SPAN-1. DUPAN-2 is considered useful for CA19-9 false-negative cases due to negativity to the Lewis-type blood group antigen. However, all of these markers have low detection performance for early pancreatic cancer, and the development of new biomarkers that contribute to treating pancreatic cancer is eagerly desired.

It was confirmed that the apolipoprotein A2 (apoA2) isoform has a lower amount in the blood in patients with early pancreatic cancer than in healthy subjects, and the possibility of detecting stage I and stage II pancreatic cancer with higher accuracy than CA19-9 has been reported (Non-Patent Literature 1). Large-scale clinical research for clinical application is underway.

Tissue factor pathway inhibitor 2 (TFPI2) is the same protein as placental protein 5 (PP5) and is a placentaderived serine protease inhibitor having three Kunitz-type protease inhibitor domains. It has been elucidated that TFPI2 is produced explicitly from the clear cell carcinoma cell line of ovarian cancer and that an increased gene expression of TFPI2 in an ovarian cancer patient tissue specifically occurs only in patients with clear cell carcinoma (Patent Literature 1). A method for detecting ovarian clear cell carcinoma by measuring TFPI2 in blood has been disclosed (Patent Literatures 2 and 3, Non Patent Literatures 2 and 3). Further, a method for detecting renal cancer by measuring the amount of TFPI2 in a sample is disclosed (Patent Literature 4).

It has elucidated that TFPI2 has undergone methylation modification to the TFPI2 DNA promoter in many cancer types, and clinical research is underway as a genetic diagnostic target using methylation modification as an indicator (Non Patent Literatures 4 to 7). As a result of investigating the methylation modification of the TFPI2 DNA promoter in various pancreatic cancer cell lines, it has been shown that full methylation, partial methylation, or non-methylation modification takes place depending on the pancreatic cancer cell line, and methylation and non-methylation of the TFPI2 DNA promoter are co-present also in pancreatic cancer tissue (Non Patent Literature 8). However, to date, it has been unclear whether the TFPI2 protein in body fluid can be applied to detect pancreatic cancer.

### PRIOR ART DOCUMENTS

### Patent Literatures

Patent Document 1: Japanese Patent 5224309
Patent Document 2: Japanese Patent 6074676
Patent Document 3: WO2016/084912
Patent Document 4: WO2019/142636

### Non Patent Literatures

Non Patent Document 1: Honda, K., et al., Scientific reports, 5, 15921.
Non Patent Document 2: J. Proteome Res., 2013, 12 (10), pp 4340-4350
Non Patent Document 3: PloS one 11.10 (2016): e0165609.
Non Patent Document 4: Takada, H., et al., Cancer genetics and cytogenetics, 197(1), 16(2010)
Non Patent Document 5: Hibi, K., et al., Anticancer research, 30(4), 1205(2010)
Non Patent Document 6: Sun, F. K., et al., Digestive diseases and sciences, 58(4), 1010(2013)
Non Patent Literature 7: Nigro, C. L., et al., Journal of Investigative Dermatology, 133(5), 1278(2013).
Non Patent Document 8: Sato, N., et al., Oncogene, 24.5, (2005):850.

### SUMMARY OF THE INVENTION

### PROBLEMS TO BE SOLVED BY THE INVENTION

An object of the present invention is to provide a method for detecting pancreatic cancer and a reagent that can be used for the method.

### MEANS FOR SOLVING THE PROBLEMS

The present inventors intensively studied, and found that blood TFPI2 is significantly increased in pancreatic cancer patients compared with healthy subjects, and arrived at an idea that TFPI2 can detect pancreatic cancer with high specificity, thereby completing the present invention.

That is, the present invention includes the following embodiments.
[1] A method for detecting pancreatic cancer, the method comprising measuring the amount of TFPI2 in a collected body fluid.
[2] The method according to [1], wherein pancreatic cancer is detected when the measured amount of TFPI2 exceeds a predetermined standard value.
[3] The method according to [1] or [2], wherein the amount of TFPI2 is the total of the amount of TFPI2 processing polypeptide and the amount of intact TFPI2.
[4] The method according to any one of [1] to [3], wherein the amount of TFPI2 is measured by an antigen-antibody reaction using an antibody that binds to an antigenic determinant in the region from the 23rd residue aspartic acid to the 131st residue histidine or the 130th residue cysteine of the amino acid sequence of SEQ ID No:1.
[5] The method according to [4], wherein the antibody is an antibody that recognizes Kunitz domain 1 of TFPI2.
[6] The method according to any one of [1] to [5], wherein measurement is carried out using mass spectrometry.
[7] The method according to any one of [1] to [6], wherein the method further comprises measuring the amount of a pancreatic cancer marker and/or a pancreatic enzyme other than TFPI2 in the body fluid.
[8] A reagent for detecting pancreatic cancer, comprising an antibody that binds to an antigenic determinant in the region from the 23rd residue aspartic acid to the 131st residue histidine or to the 130th residue cysteine in the amino acid sequence of SEQ ID NO:1.

### EFFECT OF THE INVENTION

The present invention provides a simple and highly accurate method for detecting pancreatic cancer and a reagent that can be utilized for the method.

### BRIEF DESCRIPTION OF THE DRAWINGS

Fig. 1: The figure is a diagram showing TFPI2 measurement values in a healthy subject group, a pancreatic cancer patient group, and a benign (pancreatic cystic tumor) group in box plot. The ordinate represents the amount of TFPI2 in blood.
Fig. 2: The figure is a diagram showing a receiver operating characteristic (ROC) curve of a healthy subject group and a pancreatic cancer group. The ordinate represents the sensitivity, and the abscissa represents the 1-specificity.
Fig. 3: The figure is a diagram comparing onset sites of pancreatic cancer. The ordinate represents the amount of TFPI2 in blood.
Fig. 4: The figure is a diagram comparing gender differences of pancreatic cancer patients. The ordinate represents the amount of TFPI2 in blood.
Fig. 5: The figure is a diagram showing the correlation between TFPI2 and CA19-9 in the pancreatic cancer patient group. The ordinate represents the CA19-9 measurement value, and the abscissa represents the TFPI2 measurement value.
Fig. 6: The figure is a diagram showing the correlation between TFPI2 and CA19-9 in the pancreatic cancer patient group. The ordinate represents the CA19-9 measurement value, and the abscissa represents the TFPI2 measurement value (●: Stage 1 case; ▲: Stage 2 and 3 cases).
Fig. 7: The figure is a diagram showing the correlation between TFPI2 and CEA in the pancreatic cancer patient group. The ordinate represents the CEA measurement value, and the abscissa represents the TFPI2 measurement value (●: Stage 1 case; ▲: Stage 2 and 3 cases).
Fig. 8: The figure is a diagram showing the correlation between TFPI2 and SPan-1 in the pancreatic cancer patient group. The ordinate represents the SPan-1 measurement value, and the abscissa represents the TFPI2 measurement value (●: Stage 1 case; ▲: Stage 2 and 3 cases).
Fig. 9: The figure is a diagram showing the correlation between TFPI2 and DUPAN-2 in the pancreatic cancer patient group. The ordinate represents the DUPAN-2 measurement value, and the abscissa represents the TFPI2 measurement value (●: Stage 1 case; ▲: Stage 2 and 3 cases).

### MODE FOR CARRYING OUT THE INVENTION

### <1> Method for Detecting Pancreatic Cancer of Present Invention

A first aspect of the present invention is a method for detecting pancreatic cancer, which includes measuring the amount of TFPI2 in a sample. This is a method based on the fact that TFPI2 is increasingly present in a pancreatic cancer patient's biological sample such as blood as compared with a healthy subject. The measurement of the amount of TFPI2 in a sample is usually performed in vitro. By this method, pancreatic cancer can be detected with high accuracy, as shown in the Examples described below. In particular, the method of the present invention has excellent detection performance for non-advanced pancreatic cancer (Stages 1 to 3) as compared with existing pancreatic cancer markers, and it also has excellent detection performance particularly for early pancreatic cancer (Stage 1).

The method of the present invention includes up to a step of detecting pancreatic cancer, and does not include the final act of determining the diagnosis of pancreatic cancer. A doctor diagnoses pancreatic cancer or makes a treatment policy by referring to a detection result and the like by the method of the present invention.

TFPI2 measured in the present invention is not particularly limited, and may be, for example, intact TFPI2 (hereinafter, also referred to as "I-TFPI2") and/or TFPI2 processing polypeptide (hereinafter, also referred to as "NT-TFPI2") .

SEQ ID NO:1 shows an amino acid sequence based on the human TFPI2 cDNA. In SEQ ID NO:1, a signal peptide extends from the initiation methionine to the 22nd residue glycine. "Intact TFPI2" means the peptide represented by the 23rd to 235th residues in the amino acid sequence of SEQ ID NO: 1.

"NT-TFPI2" means a peptide fragment containing a Kunitz domain 1 located on the N-terminal side of intact TFPI2, as described in Patent Document 3. More specifically, NT-TFPI2 is a peptide containing at least a sequence from the 23rd residue aspartic acid to the 131st residue histidine or the 130th residue cysteine of the amino acid sequence of SEQ ID NO:1 or a peptide containing an amino acid sequence having 80% or more identity with the above-described sequence. The identity is preferably not less than 90%, more preferably not less than 95%. The polypeptide of the present invention may be a polypeptide having the amino acid sequence which is the same as the above sequence except that one or several amino acids are deleted, substituted, inserted, and/or added. The term "several" means preferably 2 to 20, more preferably 2 to 10, and still more preferably 2 to 5. Although NT-TFPI2 may also have other peptide fragments in both sides of the above sequence, it preferably does not have an antigenic determinant for an antibody that recognizes Kunitz domain 3 of TFPI2.

The sample (test sample) in the present invention is a body fluid collected from a subject. Examples of the sample include, but are not particularly limited to, blood components such as whole blood, blood cells, serum, and plasma as well as urine, cerebrospinal fluid, and ascites. Examples of the subject usually include, but are not particularly limited to, a person suspected of having pancreatic cancer and a patient diagnosed with pancreatic cancer. It is preferable to use a body fluid such as a blood component or urine as a sample because a test can be performed easily and non-invasively, and considering the ease of collecting a sample and the versatility of other test items, it is particularly preferable to use a blood component as a sample. The sample dilution ratio may be appropriately selected from undiluted to 100-fold diluted according to the type and state of a sample to be used.

In the method for detecting pancreatic cancer of the present invention, a method for detecting TFPI2 and a method for detecting other pancreatic cancer markers and/or pancreatic enzymes may be used in combination. The combination is not particularly limited. Examples of a combination of the method for detecting TFPI2 with the method for detecting other pancreatic cancer markers and/or pancreatic enzymes in the pancreatic cancer detection method of the present invention include:
(A) a method for detecting pancreatic cancer by performing a method for detecting TFPI2 and a method for detecting other pancreatic cancer markers and/or pancreatic enzymes simultaneously (in parallel) or separately for a sample to be measured;
(B) a method for detecting pancreatic cancer by a method in which a method for detecting TFPI2 is first applied to a sample to be measured and then other pancreatic cancer markers and/or pancreatic enzymes are detected in a sample that has been determined to be negative as a result of the detection; and
(C) a method for detecting pancreatic cancer by a method in which a method for detecting other pancreatic cancer markers and/or pancreatic enzymes is first applied to a sample to be measured, and then TFPI2 is detected in a sample that has been determined to be negative as a result of the detection. The method (B) or (C) is preferable in that the reagent used for detection is not wasted. The method (A) is more preferable in that pancreatic cancer can be detected quickly.

It should be noted that detecting TFPI2, other pancreatic cancer markers, or pancreatic enzymes here involves measuring their amounts.

The other pancreatic cancer markers detected herein may be appropriately selected from, for example, conventionally known markers. Examples thereof include cancer antigen 19-9 (CA19-9) and human pancreatic cancer-related antigen (Span-1), human cancer-related sugar chain antigen (DUPAN-2), cancer antigen 50 (CA50), and carcinoembryonic antigen (CEA). Further, the pancreatic enzyme detected herein may be appropriately selected from, for example, conventionally known markers. Examples thereof include amylase (pancreatic amylase), elastase, lipase, and trypsin. Of these, CA19-9, which is most widely used as a pancreatic cancer marker, is particularly preferable as the pancreatic cancer marker used herein because its clinical usefulness has been established. Further, the tumor marker or pancreatic enzyme detected herein may be only one kind, or two kinds or more. From the viewpoint of being able to detect early pancreatic cancer, it is preferable to detect a combination of TFPI2 and CA19-9 or SPan-1. Meanwhile, from the viewpoint that pancreatic cancer which cannot be detected by other markers can be detected, it is more preferable to detect pancreatic cancer by further adding DUPAN-2 to a combination of TFPI2 and CA19-9 or SPan-1.

The sampling time of the sample in the present invention is not particularly limited. For example, a sample can be collected at any time from a preoperative period when pancreatic cancer is suspected due to clinical symptoms such as jaundice or image diagnosis or the like and detailed examination is performed to a follow-up period after definitive diagnosis of pancreatic cancer by biopsy or postoperative pathological examination, and any sample collected at any stage such as before and after definitive diagnosis and before and after the start of treatment can be used in the method of the present invention.

In the detection method of the present invention, it is preferable to determine that pancreatic cancer is detected when the amount of TFPI2 obtained by the measurement exceeds a preset standard value (Cutoff value). Here, the TFPI2 amount may be either an intact TFPI2 amount, an NT-TFPI2 amount, or a total of an intact TFPI2 amount and an NT-TFPI2 amount, and the total of the intact TFPI2 amount and the NT-TFPI2 amount is more preferable from the viewpoint of achieving both easy measurement and sufficient sensitivity and specificity.

The standard value used for the determination may be either the measurement value or the converted concentration value. The converted concentration value means a value converted from the measurement value based on a calibration curve prepared using TFPI2 as a standard sample.

The standard value (Cutoff value) for determining pancreatic cancer can be appropriately set to a measurement value showing optimum sensitivity and specificity by measuring healthy subjects and pancreatic cancer patients and analyzing a receiver operating characteristic (ROC) curve. For example, the standard value (Cutoff value) of TFPI2 may be set to 200 pg/mL as shown in the Examples described below, but is not limited thereto.

Hereinafter, a method for measuring TFPI2 will be described.

In the present invention, the amount of NT-TFPI2 or the amount of intact TFPI2 in a sample may be individually measured, or the values may be summed to obtain the total amount. The total amount of NT-TFPI2 and intact TFPI2 in a sample may be measured with a measurement system capable of measuring at one time. Alternatively, as described later, the amount of NT-TFPI2 may be indirectly measured from the total amount of both measurements and the measured amount of intact TFPI2 alone.

In the method of the present invention, the method for measuring the amount of NT-TFPI2 and/or the amount of intact TFPI2 is not limited. Examples of the method include methods utilizing antigen-antibody reaction in which an antibody that recognizes NT-TFPI2 and/or intact TFPI2 is used, and methods utilizing mass spectrometry.
(a) A competition method using a labeled measuring object and an antibody that recognizes the measuring object, which method utilizes competitive binding of the labeled measuring object and the measuring object contained in the sample to the antibody.
(b) A method using surface plasmon resonance, wherein the sample is brought into contact with a chip on which an antibody that recognizes the measuring object is immobilized, and a signal dependent on binding of the antibody to the measuring object is detected.
(c) A fluorescence polarization immunoassay using a fluorescently labeled antibody that recognizes a measuring object, which immunoassay utilizes the phenomenon that binding of the antibody to the measuring object causes an increase in the degree of fluorescence polarization.
(d) A sandwich method using two kinds of antibodies (one of which is a labeled antibody) that recognize the measuring object at different antigenic determinants, wherein formation of a complex of the three molecules, that is, the two antibodies and the measuring object, is allowed to occur.
(e) A method in which pretreatment is carried out by concentrating the measuring object in the sample using an antibody that recognizes the measuring object, and the polypeptide in the bound protein is detected using a mass spectrometer or the like.

Although the methods (d) and (e) are simple and versatile, the method (d) is preferable for processing of a large number of samples since the technologies related to the reagents and the devices for this method have been sufficiently established.

Specific examples of the methods for measuring the amount of NT-TFPI2 and/or the amount of intact TFPI2 utilizing antigen-antibody reaction include the following.
(A) A method using an antibody that recognizes both NT-TFPI2 and intact TFPI2, wherein the total amount of NT-TFPI2 and intact TFPI2 is measured (NT+I-TFPI2 assay system). The antibody that recognizes both NT-TFPI2 and intact TFPI2 is preferably an antibody that binds to an antigenic determinant in the region from the 23rd residue aspartic acid to the 131st residue histidine or to the 130th residue cysteine in the TFPI2 amino acid sequence represented by SEQ ID NO:1. The antibody is more preferably an antibody having an antigenic determinant in Kunitz domain 1 of TFPI2. In cases where the above-mentioned sandwich method is used in this method, two kinds of antibodies for different antigenic determinants are used as the antibody.
(B) A method using an antibody that does not recognize NT-TFPI2 but recognizes intact TFPI2, wherein the amount of intact TFPI2 alone is measured (I-TFPI2 assay system). The antibody that does not recognize NT-TFPI2 but recognizes intact TFPI2 is preferably an antibody that has an antigenic determinant in Kunitz domain 3 of TFPI2. In cases where the above-mentioned sandwich method is used in this method, two kinds of antibodies for different antigenic determinants are used as the antibody. At least one of these is an antibody that does not recognize NT-TFPI2 but recognizes intact TFPI2, and the other may be either an antibody that does not recognize NT-TFPI2 but recognizes intact TFPI2, or an antibody that recognizes both NT-TFPI2 and intact TFPI2.
(C) A method in which the amount of intact TFPI2 alone measured in the I-TFPI2 assay system of (B) is subtracted from the total amount of NT-TFPI2 and intact TFPI2 measured in the NT+I-TFPI2 assay system of (A), to calculate the amount of NT-TFPI2 alone.
(D) A method using an antibody that does not recognize intact TFPI2 but recognizes NT-TFPI2, wherein the amount NT-TFPI2 alone is measured. Examples of the antibody that does not recognize intact TFPI2 but recognizes NT-TFPI2 include antibodies that specifically recognize a peptide sequence in the C-terminal portion of NT-TFPI2. For example, in cases where the above-mentioned sandwich method is used, such an antibody is used as the solidphase antibody, and an antibody having an antigenic determinant in Kunitz domain 1 is used as the detection antibody.

In the method for detecting pancreatic cancer of the present invention, the amount of NT-TFPI2 alone measured by the method of (C) or (D) may be used as a criterion. However, sufficient sensitivity and specificity can be obtained also by using the total amount of NT-TFPI2 and intact TFPI2 measured by the method of (A) as a criterion. The latter method is preferable from the viewpoint of the fact that the antibody can be easily obtained, and that the measurement can be simply carried out by a single step.

An antibody that recognizes NT-TFPI2 and/or intact TFPI2 can be obtained by immunizing an animal using as an immunogen, for example, an NT-TFPI2 polypeptide or protein, an oligopeptide composed of a partial region of the intact TFPI2 polypeptide or the TFPI2 protein, or a polynucleotide encoding the intact or a partial region of the NT-TFPI2 polypeptide or of the TFPI2 protein. The protein or the oligopeptide or polypeptide may not reflect the three-dimensional structure of TFPI2 in a living body, or the structure thereof may change during a preparation process. Therefore, an obtained antibody may not have high specificity or binding capacity to TFPI2 in a desired living body, and even when a measurement system is constructed using the present antibody, the TFPI2 concentration contained in a sample as a result may not be accurately quantified.

On the other hand, when an expression vector containing a polynucleotide encoding an intact or partial region of a TFPI2 polypeptide or an intact TFPI2 protein is used as an immunogen, the intact or partial region of the TFPI2 polypeptide or intact TFPI2 protein is expressed in an immunized animal, an immune response is elicited, and an antibody having high specificity and binding capacity (that is, high affinity) to the TFPI2 in a sample is obtained, which is preferable.

The animal to be used for the immunization is not limited as long as the animal has ability to produce antibodies. The animal may be a mammal normally used for immunization, such as a mouse, a rat, or a rabbit, or may be a bird such as a chicken.

In blood, there is also TFPI1, known as a homologue of TFPI2. Therefore, it is desirable to use an antibody that does not cross with TFPI1 but specifically recognizes only TFPI2.

Another aspect of the present invention is use of a reagent for measuring the amount of TFPI2 in manufacturing of a reagent for detecting pancreatic cancer. Here, the reagent for measuring the amount of TFPI2 is preferably a reagent for measuring the total of the amount of TFPI2 processing polypeptide and the amount of intact TFPI2. The reagent for measuring the amount of TFPI2 is preferably an antibody that binds to an antigenic determinant in a region from the 23rd residue aspartic acid to the 131st residue histidine or the 130th residue cysteine of the amino acid sequence of SEQ ID NO:1, and more preferably an antibody that recognizes Kunitz domain 1 of TFPI2.

Therefore, the present invention can be also said to be use of an antibody that binds to an antigenic determinant in a region from the 23rd residue aspartic acid to the 131st residue histidine or the 130th residue cysteine of the amino acid sequence of SEQ ID NO:1 in manufacturing of a reagent for detecting pancreatic cancer.

The present invention can also be said to be the use of an antibody that binds to an antigenic determinant in a region from the 23rd residue aspartic acid to the 131st residue histidine or the 130th residue cysteine of the amino acid sequence of SEQ ID NO:1 in the detection of pancreatic cancer.

The antibody that recognizes TFPI2 may be either a monoclonal antibody or a polyclonal antibody. The antibody is preferably a monoclonal antibody.

The method of establishment of a hybridoma cell that produces an antibody that recognizes TFPI2 may be appropriately selected from methods whose techniques have been established. For example, a hybridoma cell that produces a monoclonal antibody that recognizes TFPI2 can be established by collecting B cells from an animal immunized by the above method, fusing the B cells with myeloma cells electrically or in the presence of polyethylene glycol, selecting a hybridoma cell that produces a desired antibody using HAT medium, and preparing the selected hybridoma cell into a monoclone by the limiting dilution method.

The selection of the monoclonal antibody that recognizes TFPI2 used in the present invention may be carried out based on, for example, affinity to GPI (glycosylphosphatidylinositol)-anchor type TFPI2 or secretory TFPI2 derived from a host expression system.

The host is not limited, and may be appropriately selected from microorganisms such as E. coli or yeast, insect cells, and animal cells that are usually used for protein expression by those skilled in the art. The host is preferably a mammalian cell since it enables expression of a protein having a structure similar to that of natural TFPI2 by post-translational modification such as disulfide bonding or glycosylation. Examples of the mammalian cell include the human embryonic kidney (HEK)-derived 293T cell line, monkey kidney COS7 cell line, Chinese hamster ovary (CHO) cells, and cancer cells isolated from human.

The method of purification of the antibody to be used in present invention may be appropriately selected from methods whose techniques have been established. For example, after culturing hybridoma cells which are established by the above method and which produce an antibody, the culture supernatant may be collected, and the antibody may be concentrated, if necessary, by ammonium sulfate precipitation. Thereafter, by affinity chromatography using a carrier to which Protein A, Protein G, Protein L, or the like is immobilized, and/or by ionexchange chromatography, purification of the antibody is possible.

The labeled antibody used for the antigen-antibody reaction in the sandwich method described above may be prepared by labeling an antibody purified by the above method with an enzyme such as peroxidase or alkaline phosphatase. The labeling may also be carried out using a method whose technique has been sufficiently established.

The method for measuring the amount of TFPI2 utilizing mass spectrometry in the method of the present invention is described below concretely.

In cases of a blood sample, a pretreatment step is preferably carried out by removing major proteins contained in large amounts in blood such as albumin, immunoglobulin, and transferrin using Agilent Human 14 or the like, and performing further fractionation by ion exchange, gel filtration, reverse-phase HPLC, and/or the like. Alternatively, only TFPI2 can be specifically recovered by an immunological method using an anti-TFPI2 antibody.

The measurement can be carried out by tandem mass spectrometry (MS/MS), liquid chromatography-tandem mass spectrometry (LC/MS/MS), matrix assisted laser desorption ionization time-of-flight mass spectrometry (MALDI-TOF/MS), surface enhanced laser desorption ionization mass spectrometry (SELDI-MS), or the like.

### <2> Reagent for Detecting Pancreatic Cancer of Present Invention

In cases where the reagent of the present invention is used in the sandwich method described above, the reagent preferably contains, as the antibody, two kinds of antibodies for different antigenic determinants.

The antibody contained in the reagent of the present invention may be an antibody itself, a labeled antibody, or an antibody immobilized on a solid phase.

The reagent of the present invention is described below concretely for cases where it is used for a two-step sandwich method, which is one mode of the sandwich method. However, the present invention is not limited thereto. The reagent of the present invention can be prepared by the method described in the following (I) to (III).
(I) First, Antibody 1, one of the two kinds of antibodies for different antigenic determinants used in the sandwich method that recognize TFPI2 (hereinafter referred to as "Antibody 1" and "Antibody 2"), is bound to a carrier capable of B/F (Bound/Free) separation such as an immunoplate or magnetic particles. The binding method may be either physical binding utilizing hydrophobic bonding, or chemical bonding using a linker reagent capable of cross-linking two substances to each other.
(II) After the binding of the Antibody 1 to the carrier, the carrier surface is subjected to blocking treatment using bovine serum albumin, skim milk, a commercially available immunoassay blocking agent, chemically synthesized polymer for suppressing protein adsorption, or the like for preventing non-specific binding, to provide a primary reagent.
(III) After labeling the other antibody, Antibody 2, a solution containing the obtained labeled antibody is provided as a secondary reagent. Preferred examples of the substance with which Antibody 2 is labeled include enzymes such as peroxidase and alkaline phosphatase; substances detectable with detection devices, such as fluorescent substances, chemiluminescent substances, and radioisotopes; and substances to which another molecule specifically binds, such as biotin, to which avidin specifically binds. Preferred examples of the solution for the secondary reagent include buffers with which antigen-antibody reaction can be favorably carried out, such as phosphate buffer and Tris-HCl buffer. The thus prepared reagent of the present invention may be freezedried, if necessary.

In cases of a one-step sandwich method, binding of Antibody 1 to the carrier and subsequent blocking treatment may be carried out in the same manner as in (I) and (II) to prepare an antibody-immobilized carrier, and a buffer containing a labeled Antibody 2 may be further added to the antibody-immobilized carrier, to provide a reagent.

For measurement of TFPI2 by a two-step sandwich method using reagents obtained by the method described above, the method described in the following (IV) to (VI) may be carried out.

(IV) The primary reagent prepared in (II) is brought into contact with a sample for a predetermined period of time at a constant temperature. In terms of the reaction conditions, the reaction may be carried out at a temperature within the range of 4°C to 40°C for 5 minutes to 180 minutes.

(V) Unreacted substances are removed by B/F separation, and then the secondary reagent prepared in (III) is brought into contact with the resulting reaction product for a predetermined period of time at a constant temperature to allow formation of a sandwich complex. In terms of the reaction conditions, the reaction may be carried out at a temperature within the range of 4°C to 40°C for 5 minutes to 180 minutes.

(VI) Unreacted substances are removed by B/F separation, and the labeling substance of the labeled antibody is quantified. Based on a calibration curve prepared using a TFPI2 solution having a known concentration as a standard sample, the human TFPI2 in the sample is quantified.

The amount of each reagent component such as the antibody contained in the reagent of the present invention may be appropriately set depending on conditions such as the amount of the sample, the type of the sample, the type of the reagent, and the measurement method. More specifically, for example, in cases where the amount of TFPI2 are measured as described below by a sandwich method using 20 µL of serum or plasma as a sample, the amount of the antibody to be bound to the carrier may be 100 ng to 1000 µg, and the amount of the labeled antibody may be 2 ng to 20 µg per the reaction system in which 20 µL of the sample is reacted with the antibodies.

The reagent of the present invention is applicable to either manual measurement or measurement using an automatic immunodiagnostic device. Measurement using an automatic immunodiagnostic device is especially preferable since it enables the measurement without being influenced by endogenous measurement-inhibiting factors and competing enzymes contained in the sample, and also enables rapid quantification of the concentration of TFPI2 in the sample.

The method for detecting pancreatic cancer of the present invention can be applied to the method for treating pancreatic cancer. That is, according to the present invention, provided is a method for treating pancreatic cancer in a test subject, including:
(i) a step in which a test subject is identified as one in which a measured value of the TFPI2 amount exceeds a preset standard value; and
(ii) a step in which a treatment is provided to the test subject identified as having a measured amount of TFPI2 above a preset standard value.

In a preferred aspect of the method for treating pancreatic cancer, the amount of TFPI2 is the total of the amount of TFPI2 processing polypeptide and the amount of intact TFPI2.

In the identification of the step (i), the amount of TFPI2 is preferably measured by an antigen-antibody reaction using an antibody that binds to an antigenic determinant in the region from the 23rd residue aspartic acid to the 131st residue histidine or the 130th residue cysteine of the amino acid sequence of SEQ ID No:1. More preferably, the antibody is an antibody that recognizes Kunitz domain 1 of TFPI2.

In the identification of the step (i), the measurement of the amount of TFPI2 may be performed by mass spectrometry.

Examples of the treatment of the step (ii) include surgical resection, drug therapy, and radiation therapy, and examples of the drug include tyrosine kinase inhibitors, mTOR inhibitors, and immune checkpoint inhibitors, but are not particularly limited.

### EXAMPLES

Examples are shown below for concrete description of the present invention. However, these Examples merely show examples of the present invention, and the present invention is not limited by Examples.

### <Example 1> Preparation of Reagent for TFPI2 Measurement

According to the method of Patent Document 3, a reagent for TFPI2 measurement was prepared using a TFPI2 antibody obtained by the DNA immunization method as follows.
(1) Physical adsorption of an anti-TFPI2 monoclonal antibody (TS-TF04) to water-insoluble ferrite-containing carriers was allowed at room temperature for one day and night such that the adsorption occurred at 100 ng/carrier, and blocking was then carried out with 100 mM Tris buffer (pH 8.0) supplemented with 1% BSA at 53°C for 4 hours, to prepare anti-TFPI2 antibody-immobilized carriers.
(2) An alkaline phosphatase labeled anti-TFPI2 antibody was prepared with anti-TFPI2 monoclonal antibodies (TS-TF01) using an alkaline phosphatase labeling kit (manufactured by Dojindo Laboratories).
(3) In each of magnetic force-permeable containers (volume, 1.2 mL), 12 antibody-immobilized carriers prepared in (1) were placed, and 100 µL of a buffer (Tris buffer supplemented with 3% BSA, pH 8.0) supplemented with 1 µg/mL of an alkaline phosphatase labeled antibody prepared in (2) was added thereto, followed by carrying out freezedrying, to prepare a TFPI2 assay reagent. The TFPI2 assay reagents prepared were tightly closed and sealed under nitrogen gas, and stored at 4°C until the assay.

### <Example 2> Evaluation of Clinical Samples

Table 1 shows the breakdown of the clinical samples used in this Example. Of 241 healthy subjects (102 males and 139 females), 1 pancreatic cyst tumor (benign pancreatic tumor) patient serum (male), and 23 pancreatic cancer patient sera (17 males and 6 females), samples of the cases excluding healthy males were collected by the same protocol at Yokohama City University, and were provided with informed consent and the approval of the Yokohama City University Ethics Committee. For healthy males, in-house volunteer samples that were provided with informed consent and the approval of the in-house ethics committee of Tosoh Corporation were used.

### [Table 1]

**Table 1**

| | Male | Female | Number of cases |
|---|---|---|---|
| Healthy subject | 102 | 139 | 241 |
| Benign pancreatic tumor | 1 | 0 | 1 |
| Pancreatic cancer | 17 | 6 | 23 |

As an evaluation device, a fully automatic enzyme immunoassay device AIA-2000 (manufactured by Tosoh Corporation; manufacturing/marketing notification number, 13B3X90002000009) was used. The measurement of TFPI2 by the fully automatic enzyme immunoassay device AIA-2000 was carried out by the following procedure:
(1) By automatically dispensing 20 µL of a sample and 100 µL of a diluent containing a surfactant to a container storing a TFPI2 assay reagent prepared in Example 1;
(2) carrying out antigen-antibody reaction at a constant temperature of 37°C for 10 minutes;
(3) carrying out eight times of washing using a buffer containing a surfactant after B/F separation; and
(4) adding 4-methylumbelliferyl phosphate to the container, the concentration of 4-methylumbelliferone produced by alkaline phosphatase per unit time was provided as the measurement value (TFPI2 intensity, nmol/(L·s)).

A calibration curve was prepared using a commercially available TFPI2 recombinant protein (R&D systems, Inc.) as a standard, and the TFPI2 concentration in the sample was calculated. For the blood CA19-9 values of patients with benign pancreatic tumor and patients with pancreatic cancer, the measured values described in the electronic medical records most recent on the date of blood collection were cited.

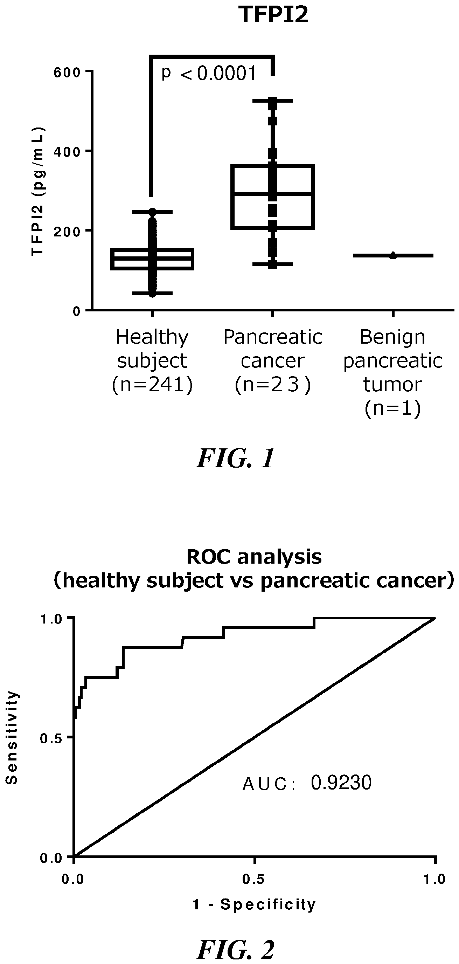

Fig. 1 shows the BoxPlot of blood TFPI2 values in healthy subjects, pancreatic cancer patients, and benign pancreatic tumor patients, and Fig. 2 shows the ROC analysis results of healthy subjects and pancreatic cancer patients. It was revealed that TFPI2 was statistically significantly higher in patients with pancreatic cancer than in healthy subjects (Mann-Whitney U test, p < 0.0001), and it was as low in patients with benign pancreatic tumor as in healthy subjects. The area under curve (AUC) calculated from the ROC curve of TFPI2 was 0.9230. From the results, it was shown that TFPI2 had favorable pancreatic cancer detection performance.

### <Example 3> Comparison of TFPI2 on the Onset Site of Pancreatic Cancer and Gender Difference

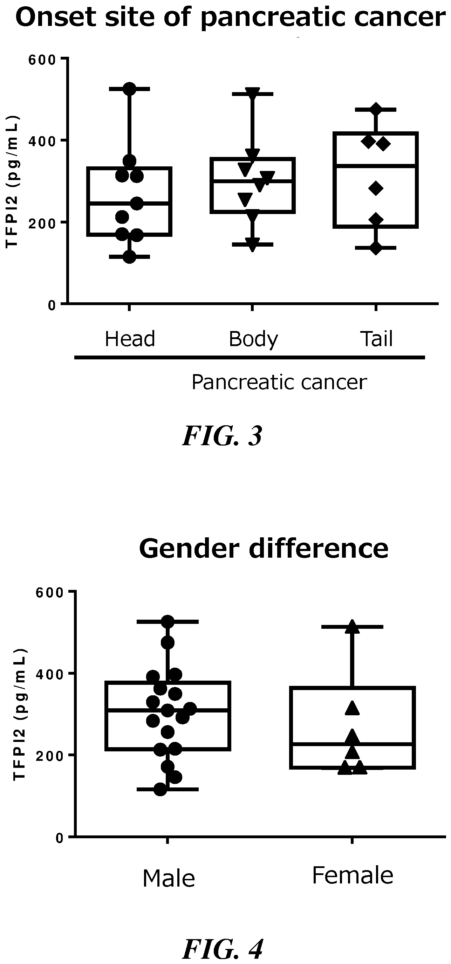

For clinical samples of Example 2, Fig. 3 shows the BoxPlot of blood TFPI2 values obtained by classifying pancreatic cancer patients into the onset sites of pancreatic cancer, which are the head, body, and tail of pancreas, and Fig. 4 shows the BoxPlot of blood TFPI2 values obtained by classifying pancreatic cancer patients based on gender difference. No difference was observed in the blood TFPI2 value between the groups depending on the onset site of pancreatic cancer and gender difference.

### <Example 4> Complementarity of TFPI2 and CA19-9 in Pancreatic Cancer Detection

For the clinical samples of Example 2, Table 2 shows the positive rates in cases where for TFPI2, 200 pg/mL, which has the maximum value of Youden index (specificity + sensitivity -1), was set as the cutoff value based on the ROC analysis result of Example 2, and for CA19-9, 37 U/mL, which is used in clinical practice, was set as the cutoff value. Although TFPI2 alone has a lower positive rate than CA19-9, it was shown that the combination of CA19-9 and TFPI2 increases the positive rate. Although CA19-9 has a problem of false-negative detection in Lewis blood group antigen-negative patients, it is expected that the positive rate of pancreatic cancer patients will be improved by measuring TFPI2 simultaneously (in parallel).

### [Table 2]

**Table 2**

| | Positive rate (%) (Breakdown of the number of cases) |
|---|---|
| CA19-9 alone ≥ 37 U/mL | 87.0 % (20/23) |
| TFPI2 alone ≥ 200 pg/mL | 79.2 % (18/23) |
| Positive for at least one of CA19-9 and TFPI2 | 95.7 % (22/23) |

### <Example 5> Correlation between TFPI2 and CA19-9 in Pancreatic Cancer Patient Sera

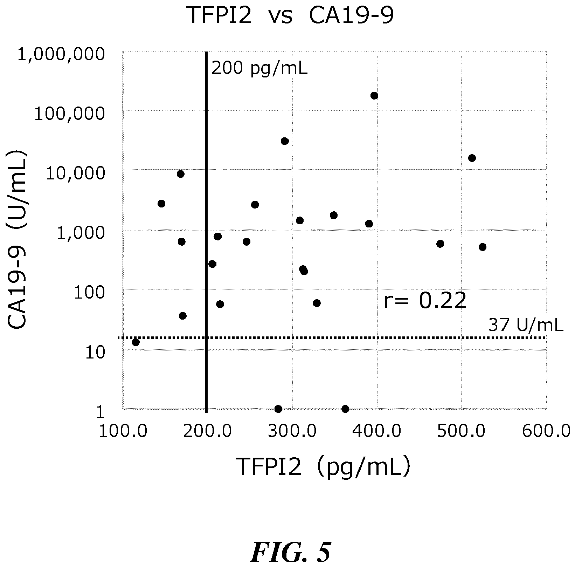

Fig. 5 shows the correlation and correlation coefficient between TFPI2 and CA19-9 in the pancreatic cancer patient sera for the clinical samples of Example 2. TFPI2 and CA19-9 were not significantly correlated (correlation coefficient = 0.22), suggesting that each is an independent indicator.

### <Example 6> Evaluation of Samples of Early Pancreatic Cancer Cases

Table 3 shows the breakdown of the clinical samples used in this Example. Twelve cases of sera from pancreatic cancer patients whose stage did not progress relatively were all samples collected by the same protocol at Yokohama City University, and were provided with informed consent and the approval of the Yokohama City University Ethics Committee.

### [Table 3]

**Table 3**

| | Number of cases | Stage1 | Stage2,3 |
|---|---|---|---|
| Non-advanced pancreatic cancer | 12 | 8 | 4 |

The TFPI2 concentration in the sample was calculated by the evaluation device and the evaluation method similar to Example 2. For the blood CA19-9, CEA, Span-1, and DUPAN-2 values of patients with pancreatic cancer, the measured values described in the electronic medical records most recent on the date of blood collection were cited.

### <Example 7> Positive Rates of Pancreatic Cancer Markers in Early Pancreatic Cancer Detection

Table 4 shows the positive rate of each pancreatic cancer marker in patients with non-advanced pancreatic cancer for the clinical samples of Example 6. The cutoff value of TFPI2 was set to 200 pg/mL as in Example 4, and the cutoff values used in clinical practice were used for other pancreatic cancer markers. The positive rate of non-advanced pancreatic cancer cases by TFPI2 was 75% (9/12 cases), indicating that it has excellent detection performance compared to other pancreatic cancer markers. In addition, the positive rate by TFPI2 was 75% (6/8 cases) even when limited to the early stage (Stage1), indicating that it has excellent detection performance as compared to other pancreatic cancer markers.

### [Table 4]

**Table 4**

| Item | Cutoff value | Positive rate (%) (Breakdown of the number of cases) | Positive rate in Stage 1 (%) (Breakdown of the number of cases) |
|---|---|---|---|
| TFPI2 | 200 pg/mL | 75.0% (9/12 cases) | 75.0% (6/8 cases) |
| CA19-9 | 37 U/mL | 25.0% (3/12 cases) | 25.0% (2/8 cases) |
| CEA | 5 ng/mL | 8.3% (1/12 cases) | 12.5% (1/8 cases) |
| Span-1 | 30 U/mL | 25.0% (3/12 cases) | 25.0% (2/8 cases) |
| DUPAN-2 | 150 U/mL | 16.7% | 12.5% |
| | | (2/12 cases) | (1/8 cases) |

### <Example 8> Complementarity between TFPI2 and Other Pancreatic Cancer Markers in Early Pancreatic Cancer Detection

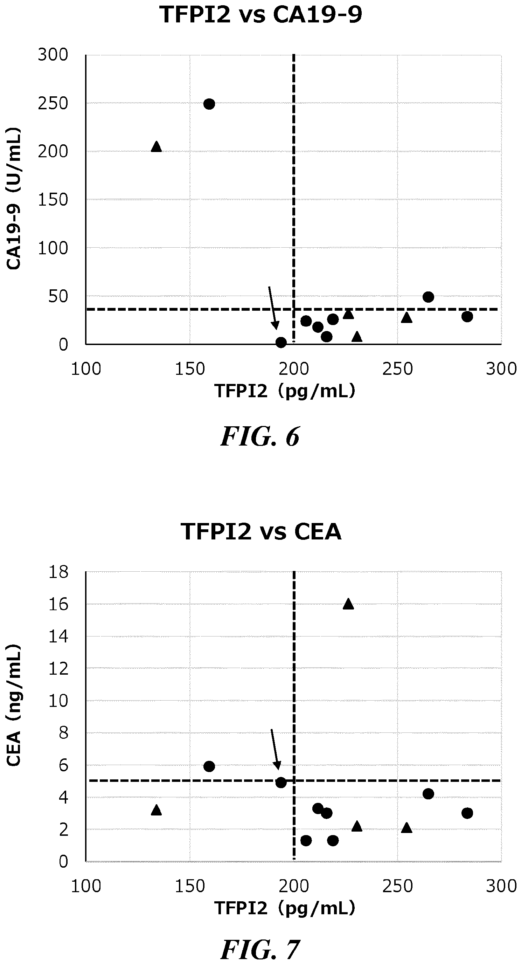

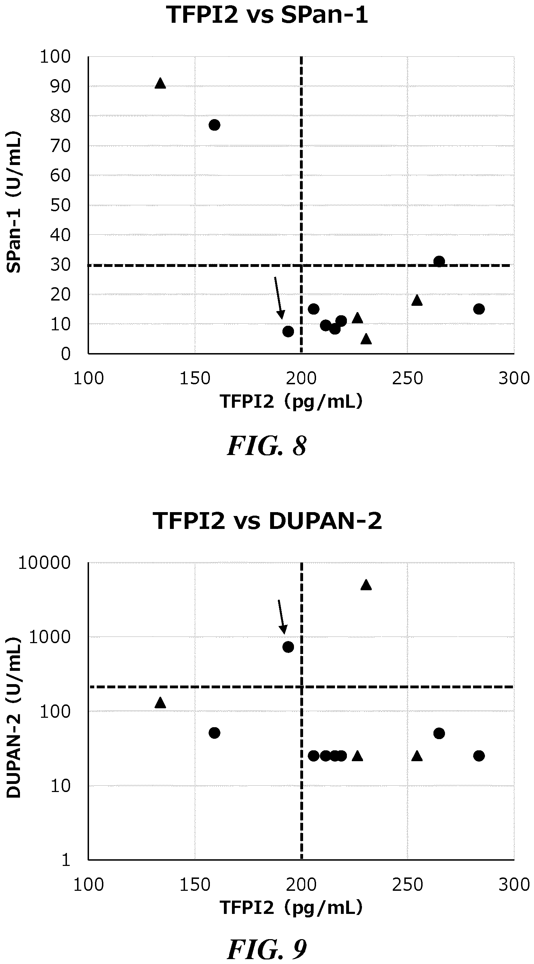

For the clinical samples of Example 6, the correlation between TFPI2 and other pancreatic cancer markers in the sera of patients with non-advanced pancreatic cancer are shown in Figs. 6 to 9 (black circles (●) indicate cases of Stage 1, and black triangles (▲) indicate cases of Stages 2 and 3, respectively, in the figures). Table 5 shows the correlation coefficient (r) between pancreatic cancer markers. No significant correlation was found between TFPI2 and other pancreatic cancer markers (r = from -0.689 to 0.068), suggesting that they are independent indicators. No correlation was generally observed between the markers other than TFPI2, but the correlation coefficient between CA19-9 and SPan-1 showed a value close to 1, and thus a high correlation was observed.

### [Table 5]

**Table 5**

| | CA19-9 | CEA | Span-1 | DUPAN-2 |
|---|---|---|---|---|
| TFPI2 | -0.689 | -0.040 | -0.680 | 0.068 |
| CA19-9 | ---- | 0.090 | 0.968 | -0.203 |
| CEA | ---- | ---- | 0.022 | -0.153 |
| Span-1 | ---- | ---- | ---- | -0.231 |

Table 6 shows the positive rates when other pancreatic cancer markers were detected alone, and the positive rates when other pancreatic cancer markers were detected in combination with TFPI2. It was confirmed that the combination of other pancreatic cancer markers with TFPI2 improved the positive rate, and in particular, the combination of TFPI2 with CA19-9 or SPan-1 showed high detection performance.

In addition, CA19-9 and SPan-1 showed similar behavior, and one case indicated by an arrow in each correlation diagram of Figs. 6 to 9 was positive only for DUPAN-2. From these facts, in the detection of patients with early pancreatic cancer, it is expected that more cases can be detected by measuring three items of pancreatic cancer markers, which are TFPI2 and DUPAN-2 plus CA19-9 or SPan-1.

### [Table 6]

**Table 6**

| Item | Positive rate (%) Marker alone , Combined with TFPI2 (Breakdown of the number of cases) | Positive rate in Stage 1 (%) Marker alone , Combined with TFPI2 (Breakdown of the number of cases) |
|---|---|---|
| CA19-9 | 25.0% → 91.7% (11/12 Cases) | 25.0% → 87.5% (7/8 Cases) |
| CEA | 8.30% → 83.3% (10/12 Cases) | 12.5% → 87.5% (7/8 Cases) |
| Span-1 | 25.0% → 91.7% (11/12 Cases) | 25.0% → 87.5% (7/8 Cases) |
| DUPAN-2 | 16.7% → 83.3% (10/12 Cases) | 12.5% → 87.5% (7/8 Cases) |

### INDUSTRIAL APPLICABILITY

The present invention provides a method for detecting a pancreatic cancer patient by a body fluid test that is simple and has a relatively low burden on the patient. This is expected to contribute to medical treatment of pancreatic cancer, which does not have an effective tumor marker and depends on image diagnosis, and is industrially considerably useful.

## Claims

1. A method for detecting pancreatic cancer, said method comprising measuring the amount of TFPI2 in a collected body fluid.

2. The method according to claim 1, wherein pancreatic cancer is detected when said measured amount of TFPI2 exceeds a predetermined standard value.

3. The method according to claim 1 or 2, wherein said amount of TFPI2 is the total of the amount of TFPI2 processing polypeptide and the amount of intact TFPI2.

4. The method according to any one of claims 1 to 3, wherein said amount of TFPI2 is measured by an antigen-antibody reaction using an antibody that binds to an antigenic determinant in the region from the 23rd residue aspartic acid to the 131st residue histidine or the 130th residue cysteine of the amino acid sequence of SEQ ID No:1.

5. The method according to claim 4, wherein said antibody is an antibody that recognizes Kunitz domain 1 of TFPI2.

6. The method according to any one of claims 1 to 5, wherein measurement is carried out using mass spectrometry.

7. The method according to any one of claims 1 to 6, wherein said method further comprises measuring the amount of a pancreatic cancer marker and/or a pancreatic enzyme other than TFPI2 in the body fluid.

8. A reagent for detecting pancreatic cancer, comprising an antibody that binds to an antigenic determinant in the region from the 23rd residue aspartic acid to the 131st residue histidine or to the 130th residue cysteine in the amino acid sequence of SEQ ID NO:1.
